# EUROPEAN PATENT APPLICATION

(11) **EP 4 438 615 A1**
(43) Date of publication of application: **02.10.2024**
(21) Application number: 22898656.8
(22) Date of filing: 25.11.2022
(51) Int. Cl.: C07K 5/11, A61K 9/127, A61K 9/51, A61K 47/24, A61K 47/28, A61K 47/34, A61K 47/42, A61K 47/46, A61K 48/00, A61P 43/00, B01J 13/02, C07K 7/00

(54) **LIPID COMPOUND, LIPOSOME, EXOSOME, LIPID NANOPARTICLE AND DRUG DELIVERY SYSTEM**

(30) Priority: 25.11.2021 JP 2021190933
(71) Applicant: NAGASAKI UNIVERSITY, Nagasaki 852-8521 (JP)
(72) Inventor: KAWAKAMI Shigeru, Nagasaki-shi, Nagasaki 852-8521 (JP); MUKAI Hidefumi, Nagasaki-shi, Nagasaki 852-8521 (JP); SUGIMOTO Yuri, Nagasaki-shi, Nagasaki 852-8521 (JP); UMINO Mizuki, Nagasaki-shi, Nagasaki 852-8521 (JP); KAMIYA Mariko, Nagasaki-shi, Nagasaki 852-8521 (JP)
(74) Representative: Cabinet Becker et Associés
(86) International application number: PCT/JP2022/043546
(87) International publication number: WO 2023/095874

(57) **Abstract**

An object of the present invention is to provide a technique capable of improving the cell binding properties of a liposome, an exosome, or a lipid nanoparticle, and a lipid compound having a group represented by Formula (1) is provided. (1): X^{a}X^{b}-(X¹)ₚ(X²)_{q}-(X³X⁴)ᵣ-(O-Z)ₛ-

## Description

### [Technical Field]

The present invention relates to a lipid compound, a liposome, an exosome, a lipid nanoparticle, and a drug delivery system.

Priority is claimed on Japanese Patent Application No. 2021-190933, filed November 25, 2021, the content of which is incorporated herein by reference.

### [Background Art]

Liposomes are artificial membranes that imitate lipid bilayers. They are about 100 to 200 nm in size and are used in drug delivery systems that encapsulate DNA, proteins, drugs, or the like in an internal water phase that delivers the contents to a specific diseased area, cosmetics, or the like.

Exosomes are vesicles secreted by cells and have a lipid bilayer of about 100 nm, and are a type of extracellular vesicle. Exosomes communicate between cells and deliver encapsulated molecules. Therefore, it is attracted attention to use as a drug delivery system.

Lipid nanoparticles contain lipids having a diameter of 10 to 1,000 nm as a main component, and are used as a drug delivery system by encapsulating a nucleic acid medicine or the like inside the particle. The main raw material of lipid nanoparticles is a lipid having biocompatibility, such as triglyceride, diglyceride, monoglyceride, fatty acids, and steroids, and a surfactant. A lipophilic molecule can be encapsulated inside the particles. In addition, lipid nanoparticles have a solid interior.

Current vaccine research has shifted from traditional vaccines (whole-killed or live-attenuated) to subunit vaccines (protein, peptide, or DNA). Subunit vaccines are much safer because they deliver only the physiologically active components necessary to generate a desirable immune response. Unfortunately, subunit vaccines are very weak immunogens, requiring delivery vehicles and the addition of immunostimulatory molecules termed adjuvants to convey protective immunity. An interesting type of delivery vehicle is peptide amphiphile micelles (PAMs), which are unique biomaterials in which the vaccine is part of the nanomaterial itself. Due to their modularity, PAMs can be readily modified to provide both vaccine antigens and adjuvants within a singular construct. Through the co-administration of a model antigenic epitope (Ovalbumin319-340-OVABT) and a known molecular adjuvant (2,3-dipalmitoyl-S-glyceryl cysteine-Pam2C, or the like), deeper insight into the mechanisms by which PAMs exert immunity activating effects has been obtained. It was found that specific combinations of an antigen and an adjuvant can significantly alter vaccine immunogenicity both in vitro and in vivo. These results show fundamental design rules that can be utilized to manufacture optimal PAM-based vaccine formulations for future disease-specific applications (Non-Patent Document 1).

Bacterial lipopeptides are strong immune modulators that activate early host responses after infection as well as initiating adjuvant effects on the adaptive immune system. These lipopeptides induce signaling in cells of the immune system through Toll-like receptor 2 (TLR2)-TLR1 or TLR2-TLR6 heteromer. So far it has been thought that triacylated lipopeptides such as the synthetic N-palmitoyl-S-[2,3-bis(palmitoyloxy)-(2RS)-propyl]-(R)-cysteine (Pam3)-CSK4 signal through TLR2-TLR1 heteromers, whereas diacylated lipopeptides such as the macrophage-activating lipopeptide signal through TLR2-TLR6 heteromers. Diacylated lipopeptides such as macrophage-activating lipopeptide derived from Mycoplasma fermentans (MALP2) and S-[2,3-bis(palmitoyloxy)-(2RS)-propyl]-(R)-cysteine (Pam2)-CGNNDESNISFKEK induce signaling through TLR2-TLR6 heteromers (Non-Patent Document 2).

It is known that, by forming a crosslinked body or a complex with a membrane-permeable peptide rich in arginine (arginine peptide), various cell membrane-impermeable physiologically active molecules including proteins can be delivered into cells. Arginine peptides are attracted to the cell surface by their interaction with proteoglycans or the like on the cell surface, and are then internalized into the cell by properly using two pathways of endocytosis and direct permeation of the plasma membrane. In addition, this uptake pathway is changed depending on the conditions such as physical properties and concentration of the crosslinked body/complex with the arginine peptide. Actin-driven liquid-phase endocytosis (macropinocytosis) is also involved in the cellular internalization of the arginine peptide by endocytosis. Membrane potential plays an important role in the direct permeation of the plasma membrane, and membrane permeation is promoted by interposing a counter-ion molecule such as pyrene butyrate (Non-Patent Document 3).

### [Citation List]

### [Non-Patent Documents]

[Non-Patent Document 1]
   Zhang, Rui, et al., "Vaccine Adjuvant Incorporation Strategy Dictates Peptide Amphiphile Micelle Immunostimulatory Capacity", The AAPS Journal, 2018, Vol. 20, Article No. 73.
[Non-Patent Document 2]
   Buwitt-Beckmann, Ute, et al., "Toll-like receptor 6-independent signaling by diacylated lipopeptides", European Journal of Immunology, 2005, Vol. 35, pp. 282 to 289.
[Non-Patent Document 3]
   Shirou Futaki, "Special Issue: Development of next-generation nanomedicine using biofunctional peptides: Intracellular migration of membrane-permeable peptides rich in arginine", Biochemistry, 2017, Vol. 89, No. 1, pp. 8 to 14.

### [Summary of Invention]

### [Technical Problem]

An object of the present invention is to provide a technique capable of improving the cell binding properties of a liposome, an exosome, or a lipid nanoparticle.

### [Solution to Problem]

The present invention includes the following aspects.
[1] A lipid compound having a group represented by Formula (1),

   (1): X^{a}X^{b}-(X¹)ₚ(X²)_{q}-(X³X⁴)ᵣ-(O-Z)ₛ-

   in Formula (1),
   X^{a} and X^{b} are each lysine (K) or arginine (R), where X^{a} and X^{b} are not arginine (R) at the same time,
   X¹ is one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   X² is one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   p and q each are 0 or 1, where p and q are each selected from 0 and 1 such that a charge of (X¹)ₚ(X²)_{q} is neutral,
   X³ is at least one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   X⁴ is at least one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   X³ and X⁴ are selected such that a charge of X³X⁴ is neutral,
   an order of X³ and X⁴ may be interchanged,
   r is an integer in a range of 1 to 8,
   Z is an alkylene group having 2 to 5 carbon atoms, and
   an average of s's is in a range of 1 to 50.
[2] The lipid compound according to [1], in which X^{a}X^{b} is KK, KR, or RK.
[3] The lipid compound according to [1] or [2], in which X³X⁴ is EK, DK, or SG, and r is an integer in a range of 3 to 5.
[4] The lipid compound according to any one of [1] to [3], in which X¹ is G, and p is 1 and q is 0.
[5] The lipid compound according to any one of [1] to [4], in which Z is an ethylene group and the average number of s' s is in a range of 1 to 10.
[6] The lipid compound according to any one of [1] to [5], in which the lipid compound is obtained by forming an ester bond, a peptide bond, or an amide bond between a hydroxy terminal of a compound represented by Formula (1') and a carboxy terminal of a lipid molecule,

   (1'): X^{a}X^{b}-(X¹)ₚ(X²)_{q}-(X³X⁴)ᵣ-(O-Z)ₛ-OH

   in Formula (1'),
   X^{a} and X^{b} are each lysine (K) or arginine (R), where X^{a} and X^{b} are not arginine (R) at the same time,
   X¹ is one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   X² is one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   p and q each are 0 or 1, where p and q are each selected from 0 and 1 such that a charge of (X¹)ₚ(X²)_{q} is neutral,
   X³ is at least one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   X⁴ is at least one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
   X³ and X⁴ are selected such that a charge of X³X⁴ is neutral,
   an order of X³ and X⁴ may be interchanged,
   r is an integer in a range of 1 to 8,
   Z is an alkylene group having 2 to 5 carbon atoms, and
   an average of s's is in a range of 1 to 50.
[7] The lipid compound according to [6], in which the lipid molecule is a lipid molecule obtained by forming an amide bond between a fatty acid and a diaminomonocarboxylic acid.
[8] The lipid compound according to [7], in which the fatty acid is a long-chain fatty acid having 13 to 21 carbon atoms.
[9] The lipid compound according to [7] or [8], in which the fatty acid is at least one selected from the group consisting of myristic acid, palmitic acid, and stearic acid.
[10] The lipid compound according to any one of [7] to [9], in which the diaminomonocarboxylic acid is lysine (L), ornithine, or homolysine.
[11] A liposome including the lipid compound according to any one of [1] to [10] contained in a lipid bilayer.
[12] An exosome including the lipid compound according to any one of [1] to [10] contained in a lipid bilayer.
[13] A lipid nanoparticle including the lipid compound according to any one of [1] to [10] contained in a lipid bilayer.
[14] A drug delivery system using the liposome according to [11].
[15] A drug delivery system using the exosome according to [12].
[16] A drug delivery system using the lipid nanoparticle according to [13].
[17] The liposome according to [11], in which the liposome is absorbed locally where the liposome has been administered, or in the vicinity thereof.
[18] The liposome according to [17], in which the local administration is to an eye, a muscle, a brain, or a visceral organ.
[19] The exosome according to [12], in which the exosome is absorbed locally where the exosome has been administered, or in the vicinity thereof.
[20] The exosome according to [19], in which the local administration is to an eye, a muscle, a brain, or a visceral organ.
[21] The lipid nanoparticle according to [13], in which the lipid nanoparticle is absorbed locally where the lipid nanoparticle has been administered, or in the vicinity thereof.
[22] The lipid nanoparticle according to [21], in which the local administration is to an eye, a muscle, a brain, or a visceral organ.

### [Advantageous Effects of Invention]

According to the present invention, it is possible to provide a technique for improving the cell binding properties of a liposome, an exosome, or a lipid nanoparticle.

### [Brief Description of Drawings]

FIG. 1 is a diagram showing an experimental method.
FIG. 2 is a diagram showing an experimental results.
FIG. 3 is a diagram showing an experimental method.
FIG. 4 is a diagram showing an experimental results.
FIG. 5 is a diagram showing an experimental method.
FIG. 6 is a diagram showing an experimental results.
FIG. 7 is a diagram showing an experimental method.
FIG. 8 is a diagram showing an experimental results.
FIG. 9 is a diagram showing an experimental method.
FIG. 10 is a diagram showing an experimental method.
FIG. 11 is a diagram showing experimental results.
FIG. 12 is a diagram showing an experimental method.
FIG. 13 is a diagram showing experimental results.
FIG. 14 is a diagram showing an experimental method.
FIG. 15 is a diagram showing experimental results.
FIG. 16 is a diagram showing an experimental method.
FIG. 17 is a diagram showing experimental results.
FIG. 18 is a diagram showing an experimental method.
FIG. 19 is a diagram showing an experimental result.
FIG. 20 is a diagram showing an experimental result.

### [Description of Embodiments]

Embodiments of the present invention will be described below. However, the embodiments described below are merely examples, and various modifications can be made to the embodiments of the present invention without departing from the spirit of the present invention.

### [Lipid compound]

The lipid compound according to the embodiments of the present invention has a group represented by Formula (1).

(1): X^{a}X^{b}-(X¹)ₚ(X²)_{q}-(X³X⁴)ᵣ-(O-Z)ₛ-.

Each symbol in Formula (1) has the following meaning.

X^{a} and X^{b} are each lysine (K) or arginine (R), where X^{a} and X^{b} are not arginine (R) at the same time.

X^{a}X^{b} is preferably KK, KR, or RK, and more preferably KK.

X¹ is one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q).

X² is lysine (K), arginine (R), or histidine (H).

p and q each are 0 or 1, where p and q are each selected from 0 and 1 such that the charge of (X¹)ₚ(X²)_{q} is neutral.

In (X¹)ₚ(X²)_{q}, it is preferably X¹ is G, and p = 1 and q = 0.

X³ is at least one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),

X⁴ is at least one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q), and

X³ and X⁴ are selected such that the charge of X³X⁴ is neutral, and the order of X³ and X⁴ may be interchanged.

X³X⁴ is preferably EK, DK, or SG, and more preferably EK.

r is an integer in the range of 1 to 8, and is preferably an integer in the range of 3 to 5 and more preferably 4.

Z is an alkylene group having 2 to 5 carbon atoms, and is preferably an ethylene group.

The average number of s' s is in the range of 1 to 50, and is preferably in the range of 1 to 10.

The lipid compound according to the embodiments of the present invention is a compound obtained by forming an ester bond, a peptide bond, or an amide bond between a hydroxy terminal of a compound represented by Formula (1') and a carboxy terminal of a lipid molecule.

(1'): X^{a}X^{b}-(X¹)ₚ(X²)_{q}-(X³X⁴)ᵣ-(O-Z)ₛ-OH

The meanings of the symbols in Formula (1') are each the same as those in Formula (1).

The lipid molecule is preferably a lipid molecule obtained by forming an amide bond between a fatty acid and a diaminomonocarboxylic acid.

The fatty acid is preferably a long-chain fatty acid having 13 to 21 carbon atoms, and more preferably at least one selected from the group consisting of myristic acid, palmitic acid, and stearic acid.

The diaminomonocarboxylic acid is preferably lysine (L), ornithine, or homolysine, and more preferably lysine.

The lipid molecule is preferably dipalmitoyllysine.

### (Liposome)

The liposome according to the embodiments of the present invention is a liposome including the above-described lipid compound contained in the lipid bilayer. That is, the lipid bilayer of the liposome is modified with the above-described lipid compound.

A method of modifying the lipid bilayer of the liposome with the above-described lipid compound is not particularly limited, but a post-modification method (post-insertion method) using a microfluidic device is preferable. The post-modification method using a microfluidic device (post-insertion method) has high reproducibility and excellent scalability, and also complies with good manufacturing practice (GMP). Furthermore, there is an advantage in that the physical properties (zeta potential and particle diameter) of the liposome are not significantly changed from those before modification.

The liposome according to the embodiments of the present invention is preferably absorbed in a local part in which the liposome has been administered, or in a vicinity of the local part, and more preferably absorbed in the locally administered.

The local part is, for example, an eye, a muscle, a joint, a bone, a brain, an abdominal cavity, or a visceral organ, but is not limited thereto. The absorption in the vicinity is not limited to a case of being close in distance, and includes, for example, absorption into a visceral organ in the case of being administered into the abdominal cavity.

### [Exosome]

The exosome according to the embodiments of the present invention is an exosome including the above-described lipid compound contained in the lipid bilayer. That is, the lipid bilayer of the exosome is modified with the above-described lipid compound.

A method of modifying the lipid bilayer of the exosome with the above-described lipid compound is not particularly limited, but a post-modification method (post-insertion method) using a microfluidic device is preferable. The post-modification method using a microfluidic device (post-insertion method) has high reproducibility and excellent scalability, and also complies with good manufacturing practice (GMP). Furthermore, there is an advantage in that the physical properties (zeta potential, particle diameter, PDI value) of the exosome are not significantly changed from those of a natural exosome.

The exosome according to the embodiments of the present invention is preferably absorbed in a local part in which the exosome has been administered, or in a vicinity of the local part, and more preferably absorbed in the locally administered.

The local part is, for example, an eye, a muscle, a bone, a joint, the brain, the abdominal cavity, or a visceral organ, but is not limited thereto. Absorption in the vicinity is not limited to a case of being close in distance, and includes, for example, absorption into a visceral organ in the case of being administered into the abdominal cavity.

### [Lipid nanoparticle]

The lipid nanoparticle according to the embodiments of the present invention is a lipid nanoparticle including the above-described lipid compound contained in the lipid membrane. That is, the lipid membrane of the lipid nanoparticle is modified with the above-described lipid compound.

A method of modifying the lipid membrane of the lipid nanoparticle with the above-described lipid compound is not particularly limited, but a post-modification method (post-insertion method) using a microfluidic device is preferable. The post-modification method using a microfluidic device (post-insertion method) has high reproducibility and excellent scalability, and also complies with good manufacturing practice (GMP). Furthermore, there is an advantage in that the physical properties (zeta potential, particle diameter, and PDI value) of the lipid nanoparticle are not significantly changed from those before modification.

The lipid nanoparticle according to the embodiments of the present invention is preferably absorbed in a local part in which the lipid nanoparticle has been administered, or in a vicinity of the local part, and more preferably absorbed in the locally administered.

The local part is, for example, an eye, a muscle, a joint, a bone, the brain, the abdominal cavity, or a visceral organ, but is not limited thereto. Absorption in the vicinity is not limited to a case of being close in distance, and includes, for example, absorption into a visceral organ in the case of being administered into the abdominal cavity.

### [Drug delivery system]

The drug delivery system according to the embodiments of the present invention is characterized by a drug delivery system using the above-described liposome, the above-described exosome, or the above-described lipid nanoparticle.

Since the above-described liposome, the above-described exosome, or the above-described lipid nanoparticle has high cell binding properties, efficient drug delivery is facilitated.

For example, the above-described liposome and the above-described exosome are suitable for delivering an anticancer agent having high cytotoxicity. In addition, the above-described lipid nanoparticle is useful for improving the delivery efficiency of easily degradable mRNA to a target cell.

In the present invention, "X^{a}X^{b}" in Formula (1) and Formula (1') may be referred to as a "functional molecule", and "-(X¹)ₚ(X²)_{q}-(X³X⁴)ᵣ-(O-Z)ₛ-" may be referred to as a "spacer".

### [Examples]

Hereinafter, the present invention will be described in more detail with reference to Examples. However, the Examples described later are examples of the present invention, and are not limited to the present invention.

### [Example 1: liposome]

### <Preparation of liposome>

### 1. Synthesis of derivative of KK-(EK)₄-(pal)₂ lipid

Various peptide derivatives were synthesized by a solid-phase synthesis method. The deprotection of 9-fluorenylmethyloxycarbonyl group (Fmoc) was carried out by treatment with 20% piperidine for 20 minutes, and the coupling of Fmoc-amino acid was carried out in the presence of 1-[bis(dimethylamino)methylene]-1H-benzotriazolium 3-oxide hexafluorophosphate (HBTU)/1-hydroxybenzotriazole (HOBt)/N,N-diisopropylethylamine (DIPEA) for 30 minutes. The unreacted amino group was acetylated in each step of the synthesis. After the coupling of Fmoc-Lys(Fmoc)-OH and deprotection of the Fmoc group, palmitic acid (10 equivalents) was coupled to the amino group of the Lys residue in the presence of HBTU/HOBt/DIPEA. A Kaiser test was carried out in order to confirm the completion of the reaction. The lipids were extracted from the resin by reacting the resin with trifluoroacetic acid (TFA)/triisopropylsilane (TIS)/H₂O (95/2.5/2.5) for 3 hours. The crude product was purified by RP-HPLC, the purity was analyzed, and then the molecular weight was confirmed by matrix-assisted laser desorption/ionization time-of-flight mass spectrometry (MALDI-TOF-MS).

### 2. Preparation of liposome

1,2-Distearoyl-sn-glycero-3-phosphocholine (DSPC), cholesterol, and 1,2-distearoylphosphatidylethanolamine methyl bonded polyethylene glycol-2000 (mPEG₂₀₀₀-DSPE) (60:35:5, molar ratio) were dissolved in methanol. In addition, 0.5 mol% of rhodamine-dioleoyl-phosphatidylethanolamine (rhodamine-DOPE) was added to label the lipid membrane. After forming a thin membrane by evaporation, the thin membrane was put into a desiccator and left overnight, then hydrated at 65°C in sterile water, and subjected to ultrasonic treatment for 3 minutes using a probe sonicator. The PEG-modified liposome was incubated with KK-(EK)₄ lipid micelle at 60°C for 1 hour to prepare a KK-(EK)₄/PEG liposome. 10× phosphate-buffered saline (10× PBS, pH 7.4) was added thereto to make the liposome isotonic, and then the liposome was filtered through a 0.45 µm filter.

The particle diameter, the zeta potential, and the polydispersity index (PDI) of the prepared liposome were measured using Zetasizer Nano ZS (manufactured by Malvern Panalytical Ltd.). Table 1 shows the particle diameter, the zeta potential, and the polydispersity index (PDI) of the liposome.

**[Table 1]**

| | Particle diameter [nm] | Zeta potential [mV] | Polydispersity index (PDI) |
|---|---|---|---|
| PEG liposome | 87.7 ± 5.4 | -1.0 ± 0.5 | 0.156 ± 0.060 |
| KK-(EK)₄/PEG liposome | 87.6 ± 2.4 | 0.0 ± 1.4 | 0.153 ± 0.023 |
| EK-(EK)₄/PEG liposome | 82.0 ± 8.3 | -0.5 ± 0.7 | 0.142 ± 0.011 |
| KK-(EK)₁/PEG liposome | 111.2 ± 31.5 | 0.6 ± 1.2 | 0.229 ± 0.108 |
| KK-(EK)₂/PEG liposome | 125.4 ± 55.8 | 0.4 ± 0.3 | 0.253 ± 0.174 |
| KK-(EK)₃/PEG liposome | 100.2 ± 17.2 | -0.8 ± 1.0 | 0.207 ± 0.096 |
| KK-(SG)₄/PEG liposome | 112.3 ± 9.5 | -0.2 ± 0.6 | 0.201 ± 0.064 |
| KR-(EK)₄/PEG liposome | 97.6 ± 7.3 | 0.6 ± 0.7 | 0.233 ± 0.078 |
| RK-(EK)₄/PEG liposome | 96.9 ± 12.9 | 0.5 ± 1.4 | 0.170 ± 0.046 |
| KKG-(EK)₄/PEG liposome | 96.0 ± 4.7 | 0.9 ± 0.2 | 0.158 ± 0.088 |
| KK-(DK)₄/PEG liposome | 95.1 ± 3.7 | 0.0 ± 0.8 | 0.162 ± 0.028 |

### <Evaluation and evaluation of liposome>

### 1. Evaluation of cell binding properties of liposome

The cells were seeded in a 24-well plate (2.5 × 10⁴ cells/cm²) and, after 24 hours, a serum-free culture medium containing 25 µM of liposome was added thereto. After 3 hours of incubation, the cells were washed twice with PBS, subjected to trypsinization, centrifuged (800 × g, 25°C, 3 minutes), and then re-suspended in PBS. This sample was analyzed by LSR Fortessa flow cytometry (manufactured by Becton, Dickinson and Company).

### 2. Evaluation of endocytosis pathway

The cells were seeded (2.5 × 10⁴ cells/cm²) in a 24-well plate and incubated for 24 hours. After the incubation, the cells were pre-incubated with 0.4 M sucrose, 200 µM genistein, or 50 µM 5-(N-ethyl-N-isopropyl)-amiloride (EIPA) at 4°C for 30 minutes. Furthermore, the cells were incubated at 4°C for 30 minutes in the presence of an inhibitor with 25 µM of KK-(EK)₄/PEG liposome, washed twice with PBS, then collected by trypsinization, centrifuged at 800 × g for 3 minutes, and re-suspended in PBS. This sample was analyzed by LSR Fortessa flow cytometry (manufactured by Becton, Dickinson and Company).

### 3. Observation with Confocal Laser Microscope

The cells were seeded (2.5 × 10⁴ cells/cm²) in a 35-mm glass dish, and, after 24 hours, incubated with a serum-free culture medium containing 25 µM liposome. After 3 hours, the cells were washed with PBS, incubated with 200 nM LysoTracker Red DND-99 (manufactured by Thermo Fisher Scientific Inc.) for 1 hour, and then the nucleus was stained with Hoechst stain (5 µg/mL). The cells were washed with PBS again, a phenol red-free culture medium was added thereto, and the cells were observed with an LSM710 confocal microscope (manufactured by Carl Zeiss AG).

### 4. Examination of the influence of Spacer

### (1) Materials

· Lipid compound
EK-(EK)₄-dipalmitoyllysine
KK-(EK)₄-dipalmitoyllysine
KK-(EK)i-dipalmitoyllysine
KK-(EK)₂-dipalmitoyllysine
KK-(EK)₃-dipalmitoyllysine
KK-(SG)₄-dipalmitoyllysine
· Liposome
PEG liposome
EK-(EK)₄/PEG liposome
KK-(EK)₄/PEG liposome
KK-(EK)₁/PEG liposome
KK-(EK)₂/PEG liposome
KK-(EK)₃/PEG liposome
KK-(SG)₄/PEG liposome
· Cell
Human lung cancer cell: A549 cell

### (2) Method

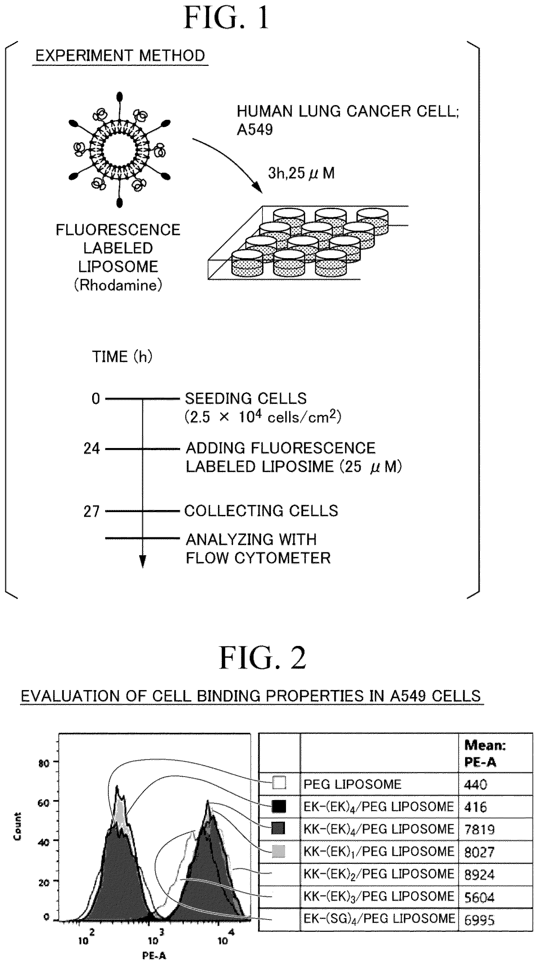

The experimental method is shown in FIG. 1.

### (3) Results

The experimental results (evaluation of cell binding properties in A549 cells) are shown in FIG. 2.

Regardless of the spacer structure, the liposome exhibited higher cell binding properties than the PEG liposome. From these findings, the possibility was shown that the KK sequence at the tip was involved in the cell binding properties of the liposome.

### 5. Examination of the influence of functional molecules

### (1) Materials

· Lipid compound
EK-(EK)₄-dipalmitoyllysine
KK-(EK)₄-dipalmitoyllysine
RK-(EK)₄-dipalmitoyllysine
KR-(EK)₄-dipalmitoyllysine
· Liposome
PEG liposome
EK-(EK)₄/PEG liposome
KK-(EK)₄/PEG liposome
RK-(EK)₄/PEG liposome
KR-(EK)₄/PEG liposome
· Cell
Human lung cancer cell: A549 cell

### (2) Method

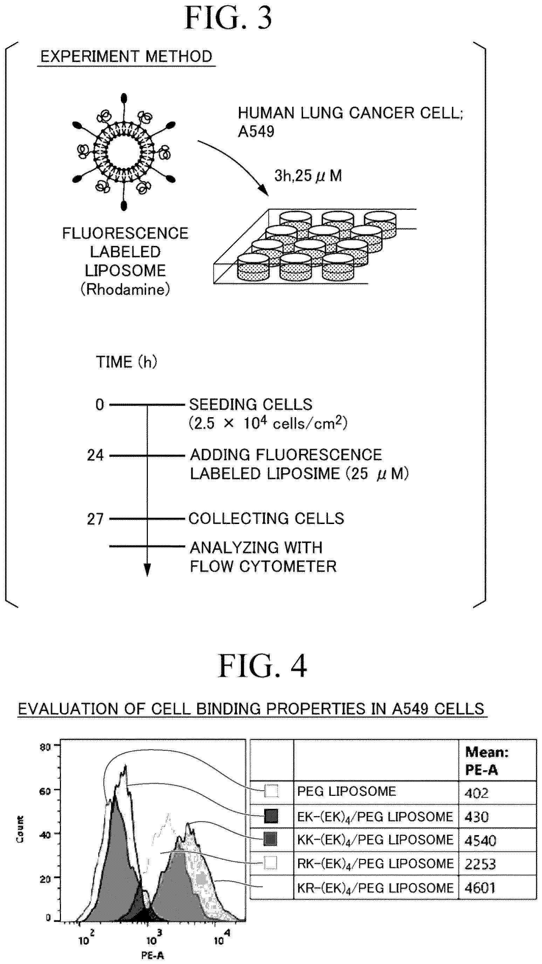

The experimental method is shown in FIG. 3.

### (3) Results

The experimental results (evaluation of cell binding properties in A549 cells) are shown in FIG. 4.

All of the liposomes exhibited higher cell binding properties than the PEG liposome, but the cell binding properties of RK-(EK)₄/PEG liposome tended to be lower than the cell binding properties of KK-(EK)₄/PEG liposome and the KR-(EK)₄/PEG liposome. From these finding, it was suggested that the presence of two cations at the tip exhibited cell binding properties. It is presumed that the difference in cell binding properties of the RK-(EK)₄/PEG liposome was also caused by the large variation in particle diameter and polydispersity index (PDI) in the evaluation of physical properties.

### 6. Examination of the influence of inserting any amino acid

### (1) Materials

· Lipid compound
EK-(EK)₄-dipalmitoyllysine
KK-(EK)₄-dipalmitoyllysine
KKG-(EK)₄-dipalmitoyllysine
· Liposome
PEG liposome
EK-(EK)₄/PEG liposome
KK-(EK)₄/PEG liposome
KKG-(EK)₄/PEG liposome
· Cell
Human lung cancer cell: A549 cell

### (2) Method

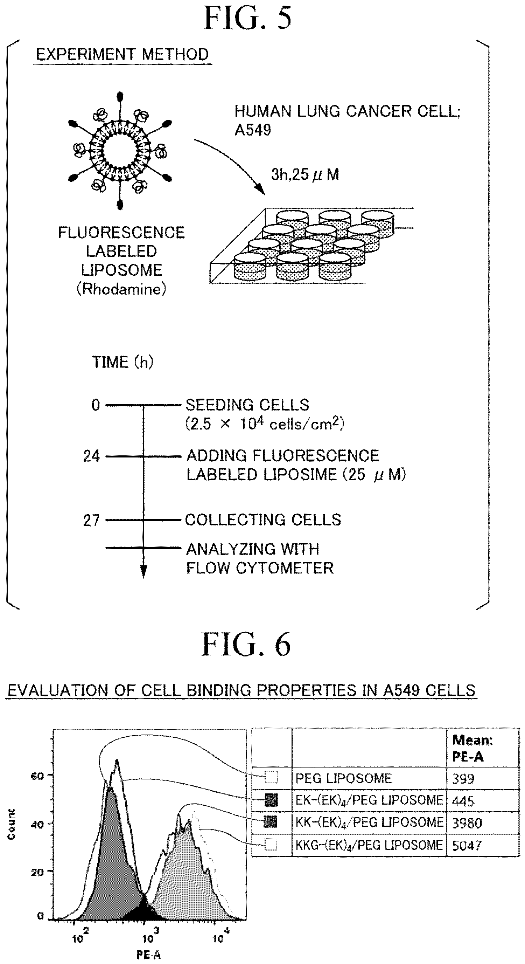

The experimental method is shown in FIG. 5.

### (3) Results

The experimental results (evaluation of cell binding properties in A549 cells) are shown in FIG. 6.

The KKG-(EK)₄/PEG liposome exhibited the same cell binding properties as that of the KK-(EK)₄/PEG liposome. From these findings, the possibility was shown that, even in the case where an amino acid was inserted between the sequence of KK at the tip and the spacer, high cell binding properties could be exhibited.

### 7. Examination of the influence of substitution of acidic amino acid (minus amino acid)

### (1) Materials

· Lipid compound
EK-(EK)₄-dipalmitoyllysine
KK-(EK)₄-dipalmitoyllysine
KK-(DK)₄-dipalmitoyllysine
· Liposome
PEG liposome
EK-(EK)₄/PEG liposome
KK-(EK)₄/PEG liposome
KK-(DK)₄/PEG liposome
· Cell
Human lung cancer cell: A549 cell

### (2) Method

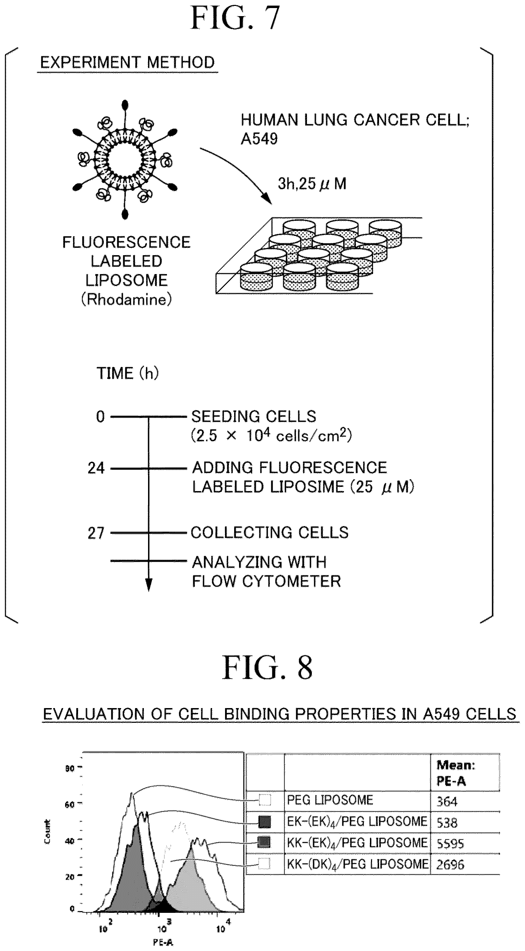

The experimental method is shown in FIG. 7.

### (3) Results

The experimental result (evaluation of cell binding properties in A549 cells) are shown in FIG. 8.

The KK-(DK)₄/PEG liposome exhibited higher cell binding properties compared with the PEG liposome, but exhibited lower cell binding properties compared with the KK-(EK)₄/PEG liposome. From these findings, it was suggested that, even in the amino acids having the same negative charge, the liposome modified with a sequence in which glutamic acid was used as the spacer structure exhibited high cell binding properties.

### [Example 2: liposome]

### <Production of liposome>

### 1. Optimization of KK-(EK)₄ structure - Production of liposome

### (1) Materials

· Lipid compound
KKKK-(EK)₄-dipalmitoyllysine
KKK-(EK)₄-dipalmitoyllysine
KK-(EK)₄-dipalmitoyllysine
K-(EK)₄-dipalmitoylly sine
EK-(EK)₄-dipalmitoyllysine
· Liposome
Rhodamine-labeled liposome (unmodified liposome)
Lipid composition (molar ratio) of DSPC:cholesterol:mPEG-DSPE = 60:35:5, 0.5 mol% Rhodamine-labeled

### (2) Method

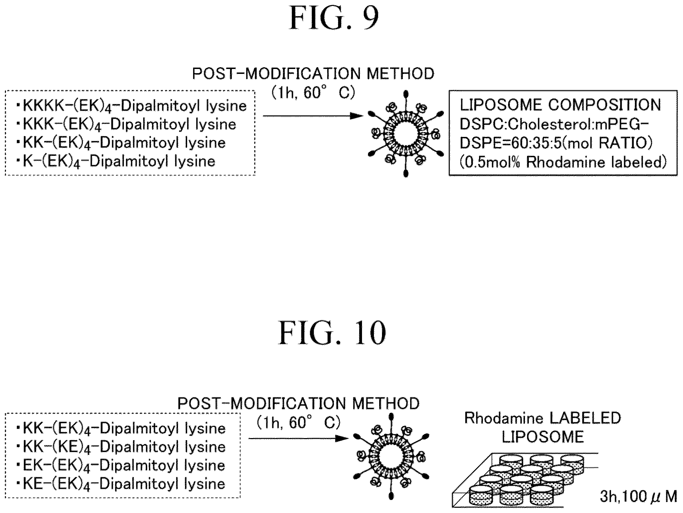

The experimental method is shown in FIG. 9.

The liposome was modified with a lipid compound by a post-modification method using NanoAssemblr (nano pharmaceutical production apparatus; manufactured by Precision NanoSystems Inc.).

The particle diameter, the zeta potential, and the polydispersity index (PDI) of the prepared liposome were measured using Zetasizer Nano ZS (manufactured by Malvern Panalytical Ltd.). Table 2 shows the particle diameter, the zeta potential, and the polydispersity index (PDI) of the liposome.

**[Table 2]**

| Modification | Particle diameter [nm] | Zeta potential [mV] | Polydisersity index (PDI) |
|---|---|---|---|
| Unmodified | 117.2 ± 1.823 | -0.614 ± 0.422 | 0.150 |
| 6% (EK)₄-EK | 119.4 ± 1.127 | -0.527 ± 0.463 | 0.106 |
| 6% (EK)₄-K | 125.2 ± 1.620 | 1.71 ± 0.215 | 0.144 |
| 6% (EK)₄-KK | 135.1 ± 0.5856 | 2.14 ± 0.406 | 0.232 |
| 6% (EK)₄-KKK | 122.7 ± 2.991 | 2.94 ± 1.17 | 0.147 |
| 6% (EK)₄-KKKK | 117.8 ± 0.4041 | 4.66 ± 0.0854 | 0.127 |

### <Examination of liposome>

### 1. Optimization of KK-(EK)₄ structure - examination of the influence of a terminal amino acid

### (1) Materials

- Lipid compound

KK-(EK)₄-dipalmitoyllysine
KK-(KE)₄-dipalmitoyllysine
EK-(EK)₄-dipalmitoyllysine
KE-(EK)₄-dipalmitoyllysine
· Liposome
Rhodamine-labeled liposome (unmodified liposome)
· Cell
Human pancreatic cancer cell: MIA Paca-2
Human lung cancer cell: A549
Mouse colon cancer cell strain: C26
Mouse fibroblast strain: NIH3T3

### (2) Method

The experimental method is shown in FIG. 10.

The liposome was modified with a lipid compound by a post-modification method using NanoAssemblr (nano pharmaceutical production apparatus; manufactured by Precision NanoSystems Inc.).

### (3) Results

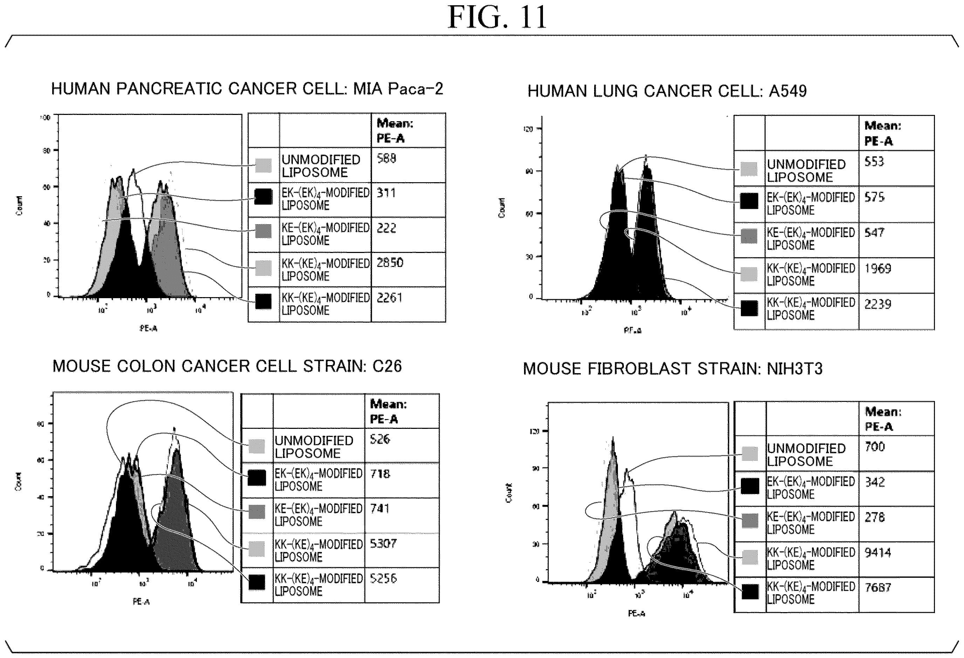

The experimental results (evaluation of cell binding properties) are shown in FIG. 11.

It was suggested that the terminal amino acid being KK was advantageous to cell binding.

### 2. Optimization of KK-(EK)₄ structure - examination of the influence of the number of terminal K

### (1) Materials

· Lipid compound
KKKK-(EK)₄-dipalmitoyllysine (referred to as "K4")
KKK-(EK)₄-dipalmitoyllysine (referred to as "K3")
KK-(EK)₄-dipalmitoyllysine (referred to as "K2")
K-(EK)₄-dipalmitoyllysine (referred to as "K1")
· Liposome
Rhodamine-labeled liposome (unmodified liposome)
· Cell
Mouse colon cancer cell strain: C26
Mouse fibroblast strain: NIH3T3

### (2) Method

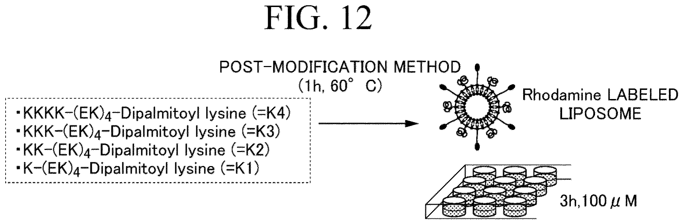

The experimental method is shown in FIG. 12.

The liposome was modified with a lipid compound by a post-modification method using NanoAssemblr (nano pharmaceutical production apparatus; manufactured by Precision NanoSystems Inc.).

### (3) Results

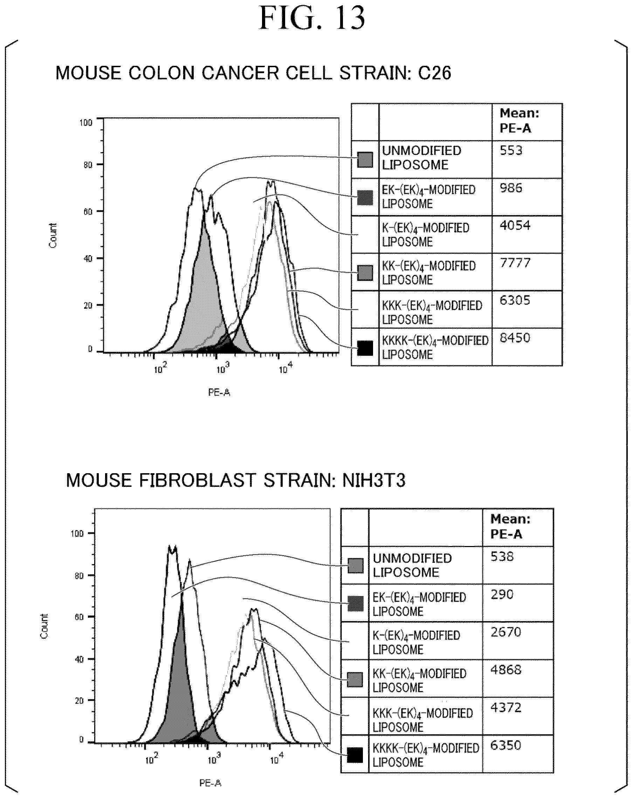

The experimental results (evaluation of cell binding properties) are shown in FIG. 13.

In the K1, K2, K3, and K4 groups, an increase in cell uptake was observed compared with the unmodified liposome.

In comparison with K1, the uptake of K2 increased in all of the cells, but the uptake of K3 and K4 when compared with K2 was the same.

From these results, it was suggested that the presence of two or more K's was advantageous to cell binding. On the other hand, it was considered that a positive charge provided little advantage.

### 3. Optimization of KK-(EK)₄ structure - examination of a terminal amino acid

### (1) Materials

· Lipid compound
KK-(EK)₄-dipalmitoyllysine (referred to as "KK-EK")
RR-(EK)₄-dipalmitoyllysine (referred to as "RR-EK")
EK-(EK)₄-dipalmitoyllysine (referred to as "EK-EK")
· Liposome
Rhodamine-labeled liposome (unmodified liposome)
· Cell
Mouse colon cancer cell strain: C26
Mouse fibroblast strain: NIH3T3

### (2) Method

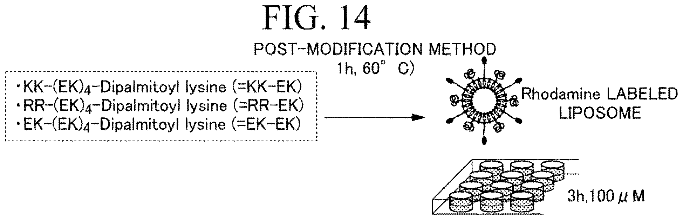

The experimental method is shown in FIG. 14.

The liposome was modified with a lipid compound by a post-modification method using NanoAssemblr (nano pharmaceutical production apparatus; manufactured by Precision NanoSystems Inc.).

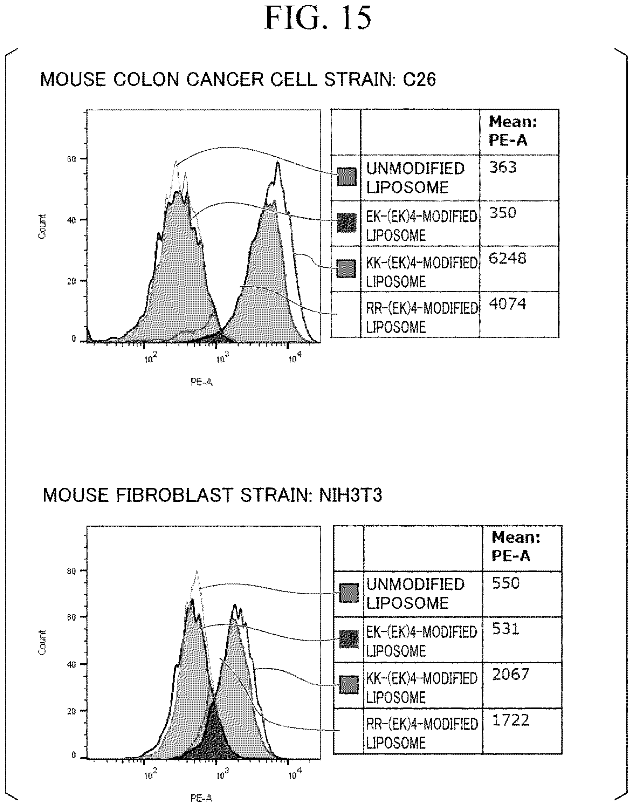

### (3) The experimental results (evaluation of cell binding properties) are shown in FIG. 15.

In the case where the liposome was modified with the RR-(EK)₄ lipid in which the tip of the peptide sequence of the KK-(EK)₄ lipid was changed to arginine (R), which is a cationic amino acid as same as lysine (K), and the cell binding properties were compared, the same cell binding properties were exhibited. From these results, it was suggested that both KK and RR exhibited the same cell binding.

### [Example 3: exosome]

### <Preparation of exosome>

### 1. Preparation of KK-(EK)₄/exosome

Exosomes were labeled with carboxyfluorescein succinimidyl ester (CFSE), and after the labeling, the protein concentration was measured using a Micro BCA protein assay kit (manufactured by Thermo Fisher Scientific Inc.). In the post-modification method in the related art, the KK-(EK)₄ lipid micelle and the exosome solution were added to each other in an amount of 1: 1 in terms of the protein level, and the mixture was incubated at 37°C for 1 hour to prepare KK-(EK)₄/exosome. In the microfluidic device method, a KK-(EK)₄ lipid micelle and an exosome solution were prepared in an amount of 1:1 in terms of the protein level, and loaded into a microfluidic chip. The preparation conditions were set to a flow rate ratio of 1:1 and a total flow rate of 1 mL/min. The particle diameter, the zeta potential, and the polydispersity index (PDI) of the prepared sample were measured using Zetasizer Nano ZS (manufactured by Malvern Panalytical Ltd.).

### <Evaluation of exosome>

### 1. Evaluation of cell binding properties of exosome

The cells were seeded in a 48-well plate (2.5 × 10⁴ cells/cm²) and, after 24 hours, a serum-free culture medium containing 5 µg/mL of exosome was added thereto. After 3 hours, the cells were washed twice with PBS, collected by trypsinization, centrifuged at 800 × g for 3 minutes, and re-suspended in PBS. This sample was measured using a LSR Fortessa flow cytometer (manufactured by Becton, Dickinson and Company).

### [Example 4: lipid nanoparticle (LNP)]

### <Preparation of LNP>

### 1. Preparation of KK-(EK)₄-modified mRNA/LNP

Dlin-MC3-DMA, DSPC, Cholesterol, and DSG-PEG₂₀₀₀ (50:10:38.5:1.5, molar ratio) were dissolved in ethanol, and KK-(EK)₄-modified mRNA/LNP was prepared to be 15 µg/mL mRNA (in 100 mM citrate buffer). As mRNA, mRNA-luciferase encoding firefly luciferase was used. The solution was loaded into a microfluidic chip at a flow rate ratio of 1:3 and a total flow rate of 12 mL/min. The prepared sample was dialyzed in PBS (pH 7.4) overnight. The sample was concentrated using Amicon, and the particle diameter and the zeta potential of LNP were measured using Zetasizer Ultra (manufactured by Malvern Instruments Ltd.), and the encapsulation rate of mRNA was measured by a RiboGreen assay. Table 3 shows the particle diameter, the zeta potential, and the mRNA encapsulation rate of the liposome.

**[Table 3]**

| Modification | Particle diameter [nm] | Zeta potential [mV] | mRNA encapsulation rate [%] |
|---|---|---|---|
| Unmodified | 108.1 | -6.576 | 96.84 |
| 1 mol% KK-(EK)₄ | 129.1 | -4.337 | 97.49 |
| 3 mol% KK-(EK)₄ | 130.7 | -5.823 | 96.21 |
| 5 mol% KK-(EK)₄ | 140.2 | -12.53 | 98.40 |

### <Evaluation of LNP>

### 1. In vitro luciferase assay of KK-(EK)₄-modified mRNA/LNP

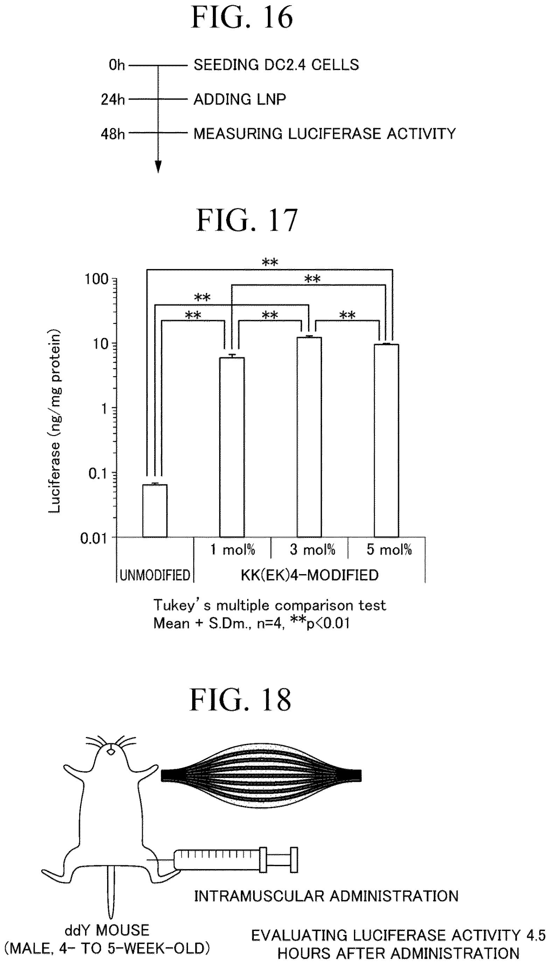

DC2.4 cells were seeded (4 × 10⁴ cells/cm²) in a 48-well plate, and after 24 hours, LNP containing 0.114 µg of mRNA was added to a Complete RPMI culture medium, and incubated with the cells. After 24 hours, the cells were washed twice with PBS, treated with Lysis buffer, and then centrifuged (20,600 × g, 4°C, 5 minutes), and the supernatant of the sample was measured with a luminometer (Luminescencer-PSN, manufactured by Atto Corporation). Furthermore, the protein level was measured using BCA Protein Assays (manufactured by Thermo Fisher Scientific Inc.). An outline of the experimental method is shown in FIG. 16, and experimental results are shown in FIG. 17.

In the DSG-PEG/LNP, the luciferase expression was maximized with 3 mol% KK-(EK)₄-modified lipid.

### 2. In vivo luciferase assay of KK-(EK)₄-modified mRNA/LNP

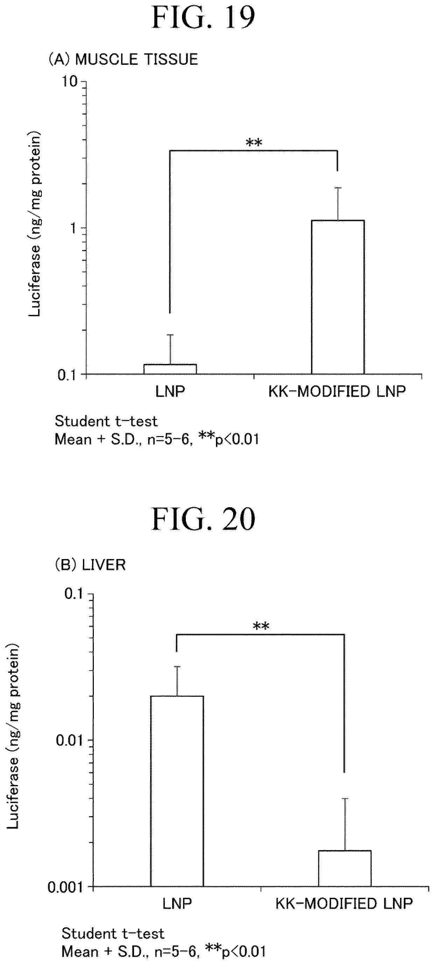

The mRNA/LNP (2 µg mRNA/mouse) was administered into the peroneal muscle of the right hind leg of a ddY mouse (male 4- to 5-week-old, Japan SLC, Japan). The blood was drawn 4.5 hours after administration, and the liver and peroneal muscle of the right hind leg as the administration site were collected. The collected tissue was washed twice with cold PBS, a dissolution buffer was added thereto, and the tissue was homogenized using a homogenizer (Omni Tissue Homogenizer, OMNI International, USA). The tissue homogenate was centrifuged (4°C, 15,000 × g, 5 minutes), and the supernatant was collected. The supernatant was added to PicaGene (Toyobo, Japan), and the luciferase activity was measured using a luminometer (ATTO-2270, manufactured by ATTO Corporation). The data were corrected on the basis of a calibration curve using a luciferase standard solution (Toyobo) and the protein concentration, which was determined by the BCA assay. As the mRNA/LNP, unmodified mRNA/LNP (referred to as "LNP") or 3mol% KK-(EK)₄-modified mRNA/LNP (referred to as "KK-modified LNP") was used. An outline of the experimental method is shown in FIG. 18, and the experimental results each are shown in FIG. 19 (muscle cell) and FIG. 20 (liver cell).

Significant increase in expression in the muscle and significant decrease in expression in the liver were confirmed by KK modification.

## Claims

1. A lipid compound having a group represented by Formula (1),
(1): X^{a}X^{b}-(X¹)ₚ(X²)_{q}-(X³X⁴)ᵣ-( O-Z)ₛ-
in Formula (1),
X^{a} and X^{b} are each lysine (K) or arginine (R), where X^{a} and X^{b} are not arginine (R) at the same time,
X¹ is one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
X² is one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
p and q are each 0 or 1, where p and q are each selected from 0 and 1 such that a charge of (X¹)ₚ(X²)_{q} is neutral,
X³ is at least one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
X⁴ is at least one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
X³ and X⁴ are selected such that a charge of X³X⁴ is neutral,
an order of X³ and X⁴ may be interchanged,
r is an integer in a range of 1 to 8,
Z is an alkylene group having 2 to 5 carbon atoms, and
an average of s's is in a range of 1 to 50.

2. The lipid compound according to Claim 1,
wherein X^{a}X^{b} is KK, KR, or RK.

3. The lipid compound according to Claim 1,
wherein X³X⁴ is EK, DK, or SG, and
r is an integer in a range of 3 to 5.

4. The lipid compound according to Claim 1,
wherein X¹ is G, and
p = 1 and q = 0.

5. The lipid compound according to Claim 1,
wherein Z is an ethylene group, and
the average of s's is in a range of 1 to 10.

6. The lipid compound according to Claim 1,
wherein the lipid compound is obtained by forming an ester bond, a peptide bond, or an amide bond between a hydroxy terminal of a compound represented by Formula (1') and a carboxy terminal of a lipid molecule,
(1'): X^{a}X^{b}-(X¹)ₚ(X²)_{q}-(X³X⁴)ᵣ-(O-Z)ₛ-OH
in Formula (1'),
X^{a} and X^{b} are each lysine (K) or arginine (R), where X^{a} and X^{b} are not arginine (R) at the same time,
X¹ is one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
X² is one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
p and q are each 0 or 1, where p and q are each selected from 0 and 1 such that a charge of (X¹)ₚ(X²)_{q} is neutral,
X³ is at least one selected from the group consisting of aspartic acid (D), glutamic acid (E), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
X⁴ is at least one selected from the group consisting of lysine (K), arginine (R), histidine (H), glycine (G), alanine (A), phenylalanine (F), leucine (L), isoleucine (I), cysteine (C), methionine (M), tyrosine (Y), valine (V), threonine (T), serine (S), proline (P), tryptophan (W), asparagine (N), and glutamine (Q),
X³ and X⁴ are selected such that a charge of X³X⁴ is neutral,
an order of X³ and X⁴ may be interchanged,
r is an integer in a range of 1 to 8,
Z is an alkylene group having 2 to 5 carbon atoms, and
an average of s's is in a range of 1 to 50.

7. The lipid compound according to Claim 6,
wherein the lipid molecule is a lipid molecule obtained by forming an amide bond between a fatty acid and a diaminomonocarboxylic acid.

8. The lipid compound according to Claim 7,
wherein the fatty acid is a long-chain fatty acid having 13 to 21 carbon atoms.

9. The lipid compound according to Claim 7,
wherein the fatty acid is at least one selected from the group consisting of myristic acid, palmitic acid, and stearic acid.

10. The lipid compound according to Claim 7,
wherein the diaminomonocarboxylic acid is lysine (L), ornithine, or homolysine.

11. A liposome comprising:
the lipid compound according to any one of Claims 1 to 10 contained in a lipid bilayer.

12. An exosome comprising:
the lipid compound according to any one of Claims 1 to 10 contained in a lipid bilayer.

13. A lipid nanoparticle comprising:
the lipid compound according to any one of Claims 1 to 10 contained in a lipid.

14. A drug delivery system using the liposome according to Claim 11.

15. A drug delivery system using the exosome according to Claim 12.

16. A drug delivery system using the lipid nanoparticle according to Claim 13.

17. The liposome according to Claim 11,
wherein the liposome is absorbed locally where the liposome has been administered, or in the vicinity thereof.

18. The liposome according to Claim 17,
wherein the local administration is to an eye, a muscle, a bone, a joint, an abdominal cavity, a brain, or a visceral organ.

19. The exosome according to Claim 12,
wherein the exosome is absorbed locally where the exosome has been administered, or in the vicinity thereof.

20. The exosome according to Claim 19,
wherein the local administration is to an eye, a muscle, a bone, a joint, an abdominal cavity, a brain, or a visceral organ.

21. The lipid nanoparticle according to Claim 13,
wherein the lipid nanoparticle is absorbed locally where the lipid nanoparticle has been administered, or in the vicinity thereof.

22. The lipid nanoparticle according to Claim 21,
wherein the local administration is to an eye, a muscle, a bone, a joint, an abdominal cavity, a brain, or a visceral organ.
